# EUROPÄISCHE PATENTSCHRIFT

(11) **EP 0 568 782 B1**
(45) Veröffentlichungstag und Bekanntmachung des Hinweises auf die Patenterteilung: **29.05.1996**
(21) Anmeldenummer: 93103261.9
(22) Anmeldetag: 02.03.1993
(51) Int. Cl.: C07C 263/04, C07C 263/06

(54) **Kontinuierliches mehrstufiges Verfahren zur Herstellung von (cyclo)-aliphatischen Diisocyanaten**
Continuous and multistep process for the preparation of (cyclo)-aliphatic diisocyanates
Procédé continu, à plusieurs stades successifs apte à la préparation de diisocyanates (cyclo)-aliphatique

(30) Priorität: 05.05.1992 DE 4214236; 19.09.1992 DE 4231417
(43) Veröffentlichungstag der Anmeldung: 10.11.1993
(73) Patentinhaber: HÜLS AKTIENGESELLSCHAFT, D-45764 Marl (DE)
(72) Erfinder: Bohmholdt, Gerd, Dr., W-4370 Marl (DE); Heitmann, Wilhelm, Dr., W-4690 Herne 1 (DE); Kirchner, Peter, W-4630 Bochum 1 (DE); Michalczak, Hans-Werner, W-4690 Herne 2 (DE)

(56) Entgegenhaltungen:
- EP-A- 0 018 586
- EP-A- 0 054 817
- EP-A- 0 126 299
- EP-A- 0 355 443
- DE-A- 3 314 790
- US-A- 2 677 698

## Beschreibung

Die Erfindung betrifft ein verbessertes mehrstufiges Verfahren zur kontinuierlichen phosgenfreien Herstellung von (cyclo)aliphatischen Diisocyanaten, das die Umwandlung Von (cyclo)aliphatischen Diaminen in die entsprechenden (Cyclo)alkylenbisharnstoffe, deren Umsetzung mit Alkohol zu den (Cyclo)alkylenbiscarbamaten und die thermische Spaltung letzterer in die (Cyclo)alkylendiisocyanate und Alkohol in flüssiger Phase umfaßt.

Diisocyanate sind wertvolle chemische Verbindungen, die nach dem Prinzip des Diisocyanat-Polyadditionsverfahrens den gezielten Aufbau von Polymeren erlauben, welche als Polyurethane oder Polyharnstoffe in Schaumstoffen, Elastomeren, Thermoplasten, Fasern, Beschichtungen oder Klebstoffen vielfältigen industriellen Einsatz finden. Die großtechnische Herstellung von Diisocyanaten erfolgt bisher über die Phosgenierung der entsprechenden Diamine, d. h. unter Verwendung von korrosivem, sehr giftigem und einen hohen Anteil an Chlor enthaltendem Phosgen.

Es sind bereits eine Reihe von Vorschlägen zur Herstellung von (cyclo)aliphatischen Isocyanaten bekannt, die die Verwendung von Phosgen umgehen, indem die den Diisocyanaten zugrundeliegenden Diamine zuerst in Biscarbamate umgewandelt und dann in einem nächsten Schritt einer thermischen Spaltung unterworfen werden. Dadurch können gravierende Umweltschutzprobleme und sicherheitstechnischer Mehraufwand vermieden und gleichzeitig chlorfreie Isocyanate erhalten werden, was sich besonders vorteilhaft bei der späteren Verwendung in Polyurethanen bemerkbar macht.

Die Herstellung von (cyclo)aliphatischen Biscarbamaten in einer Eintopfreaktion aus Harnstoff, Diamin und Alkohol bei Temperaturen von 160 bis 300 °C unter gleichzeitiger Abtrennung von Ammoniak ist aus der EP-PS 18 586 bekannt.

Die Lehre der EP-PS 18 586 wurde weiterentwickelt in den EP-PSS 126 299 und 126 300 sowie in EP-A 355 443. Nachteil der dort vorgenommenen simultanen Umsetzung von Harnstoff, Diamin und Alkohol ist die durch unvermeidbar ablaufende Nebenreaktionen im o. g. Temperaturbereich verminderte Selektivität. Wie die Beispiele zeigen, entstehen neben den gewünschten Biscarbamaten durch Reaktion von Harnstoff und Alkohol beträchtliche Anteile an N-unsubstituiertem Alkylcarbamat und Dialkylcarbonat sowie durch N,N'-Substitution des Harnstoffs durch das Diamin Polyharnstoffe. Diese Nebenprodukte müssen vor der thermischen Spaltung der Biscarbamate abgetrennt werden.

In EP-PS 27 953 wird ebenfalls ein einstufiges Verfahren zur Herstellung von Carbamaten durch Umsetzung von Harnstoff, primären Aminen und Alkoholen beschrieben, wobei die Ausbeuten durch Zusatz von ohnehin bei dieser Reaktion als Zwischenprodukte auftretenden N-unsubstituierten Carbamaten und/oder N-mono- oder N,N'-disubstituierten Harnstoffen oder Polyharnstoffen erhöht werden sollen. Wie den Beispielen zu entnehmen ist, ergibt dieses Verfahren trotzdem keine technisch befriedigenden Ausbeuten.

Gemäß EP-PS 28 331 werden u. a. Biscarbamate durch Umsetzung von linearen Polyharnstoffen mit Alkohol in Gegenwart von N-unsubstituiertem Carbamat und/oder Harnstoff hergestellt. Damit soll die Bildung von Aminen verhindert werden, welche bei der Reaktion von N,N'-disubstituierten Harnstoffen mit Alkohol entstehen. Auch hier sind die erzielten Ausbeuten technisch nicht befriedigend.

In den beiden letztgenannten Patentschriften finden sich weder Beispiele noch Hinweise für die Umsetzung von Bisharnstoffen mit Alkohol in Abwesenheit von Diamin und/oder Harnstoff.

Die Herstellung von N-monosubstituierten Carbamaten aus trisubstituierten Harnstoffen und Hydroxylverbindungen ist in DE-PS 2 258 454 beschrieben. Monocarbamate werden gemäß US-PS 2 677 698 in zwei Stufen hergestellt, wobei in erster Stufe aus Harnstoff und Amin ohne Lösungsmittel die Bildung von N,N'-disubstituierten Harnstoffen erfolgt und in zweiter Stufe unter Zusatz von Monoalkoholen die Bildung der Carbamate.

Eine kontinuierliche Herstellung von Biscarbamaten wird nur gemäß der bereits zitierten Schrift EP-PS 126 299 durchgeführt, indem eine dreistufige Rührkesselkaskade verwendet wird. Der Nachteil der Rührkesselkaskade besteht darin, daß jeweils geregelte Energiezufuhr, Kolonne zur Abtrennung des Ammoniaks und Druckhaltung für jede Stufe und damit insgesamt eine hohe Investition erforderlich ist.

Eine halbkontinuierliche Herstellung von Biscarbamaten wird in der EP-A 355 443 beschrieben, wo alternierend betriebene Rührreaktoren verwendet werden und die Aufarbeitung kontinuierlich erfolgt. Der Nachteil der alternierend betriebenen Rührreaktoren liegt im diskontinuierlichen Beschicken und Entleeren, was mit zusätzlichem bedienungstechnischen und apparativen Aufwand verbunden ist.

Die Herstellung von Bisharnstoffen aus Harnstoff und Diamin in Substanz bei 130 bis 140 °C in 3 bis 4 h mit hohen Ausbeuten ist aus der US-PS 2 145 242 und US-PS 2 445 518 bekannt. Nachteilig hierbei ist, daß unter den genannten Reaktionsbedingungen Bisharnstoffe vornehmlich als Feststoffe anfallen, wodurch für eine Weiterverarbeitung erhebliche verfahrenstechnische Probleme auftreten. Vorgeschlagen wird daher auch die Durchführung der Reaktion in Gegenwart von sich inert verhaltenden Verdünnungs-oder Lösungsmitteln. Als solche werden z. B. chlorierte Benzole, Phenole und Kresole nach US-PS 2 145 242 und JP-Sho 38-20748 oder auch Wasser nach US-PS 2 213 578 bzw. Bachmann et al., J. Am. Chem. Soc. 72 (1950), 3132 vorgeschlagen, die bei einer Weiterverarbeitung des Bisharnstoffs in der Regel wieder abgetrennt werden müssen. Die Verwendung eines primären aliphatischen Monoalkohols wird in keiner der Patentschriften erwähnt.

In der JP-Sho 38-20748 wird die Herstellung von kettenförmigen Kondensationsharzen mit Urethan- und Harnstoffbindungen beschrieben. Dabei wird in einer ersten Reaktionsstufe bei Temperaturen von 100 bis 130 °C Diamin und Harnstoff in Gegenwart von Diol zu einem festen Reaktionsprodukt umgesetzt, das neben untergeordneten Mengen des Bisharnstoffs höherkondensierte Produkte des linearen Polyharnstofftyps enthält. Letztere weisen die innenliegenden Harnstoffgruppierungen -NH-CO-NH- auf, die auch während der Kondensation zum Polyharnstoffpolyurethan bei 230 °C in Gegenwart des Diols stabil bleiben.

Ebenfalls ist aus der EP-PS 18 586 bekannt, daß die Reaktion von Harnstoff mit Hexamethylendiamin in Gegenwart von Butanol bei 120 bis 150 °C Poly-hexamethylen-harnstoff ergibt, der anschließend bei einer Temperatur von 190 °C in Butanol unlöslich ist und sich nicht in Biscarbamat überführen läßt.

Die thermische Spaltung von (cyclo)aliphatischen Biscarbamaten kann in der Gas- oder in der Flüssigphase, mit oder ohne Lösungsmittel und mit oder ohne Katalysatoren erfolgen. So werden In den EP-PSS 126 299 und 126 300 Verfahren zur Herstellung von Hexamethylendiisocyanat bzw. Isophorondiisocyanat durch Spaltung der entsprechenden Biscarbamate in der Gasphase im Rohrreaktor in Gegenwart von metallischen Füllkörpern bei 410 °C beschrieben. Abgesehen davon, daß solch hohe Temperaturen nur noch mit aufwendiger Technologie einzustellen sind, ist das Verfahren mit dem Nachteil behaftet, daß bei dieser Temperatur bereits teilweise Crackung der Reaktionsprodukte stattfindet, verbunden mit Ablagerungen auf den Füllkörpern und einem Zuwachsen des Rohrreaktors, so daß das Verfahren wegen der kurzen Standzeit für eine technische Produktion wenig geeignet ist.

Ein kontinuierliches Verfahren der Spaltung von (cyclo)aliphatischen Biscarbamaten in der flüssigen Phase in Gegenwart von Katalysatoren ohne Lösungsmittel wird in der EP-A 355 443 angegeben. Hier werden in einem Rührreaktor mit 200 g Inhalt unter intensivem Sieden des Reaktionsgemisches bei 233 °C und 27 mbar 1,5-Diisocyanato-2-methylpentan und andere (cyclo)aliphatische Diisocyanate in hoher Ausbeute hergestellt. Nachteilig ist hier, daß die bei Kapazitätserhöhung erforderliche Vergrößerung des Spaltreaktors zu einer Verringerung des Verhältnisses von Wärmeübertragungsfläche zu Inhalt führt. Bei unveränderter spezifischer Heizleistung muß die Wandtemperatur erhöht werden, was zu Zersetzungen und Anbackungen und damit zur Verschlechterung des Wärmeüberganges führt. Daher kommt auch dieses Verfahren für eine technische Produktion nicht in Betracht.

Ein weiteres kontinuierliches Verfahren zur Spaltung von (cyclo)aliphatisehen Biscarbamaten findet sich in der EP-A 323 514, wo in einem 300 ml-Kolben mit aufgesetzter Kolonne in Gegenwart von 100 g teilweise hydriertem Terphenyl und Manganacetat als Katalysator Isophorondiisocyanat in guter Ausbeute aus dem entsprechenden Biscarbamat hergestellt wird. Auch hier verschlechtert sich die Ausbeute bei einer Kapazitätserhöhung aufgrund des ungünstigeren Verhältnisses Wärmeübertragungsfläche zu Inhalt erheblich, so daß auch dieses Verfahren nicht für die technische Produktion geeignet ist.

Die EP-PS 54 817 lehrt, daß bei der kontinuierlichen Spaltung von Monocarbamaten in flüssiger Phase ohne Lösungsmittel, gegebenenfalls in Gegenwart von Katalysatoren und/oder Stabilisatoren, die Abtrennung von nicht umgesetztem Monocarbamat sowie die Isolierung der Spaltprodukte Monoisocyanat vom Alkohol nur bei Verwendung von Dephlegmatoren zu guten Ergebnissen führt, während bei Einsatz einer Destillationskolonne mit Seitenstromentnahme die Gewinnung der Spaltprodukte gar nicht oder nur mit sehr schlechter Ausbeute möglich ist. Allerdings enthalten die Kondensate aufgrund der geringen Trennleistung von Dephlegmatoren die gewünschten Komponenten Isocyanat und Alkohol nur in mäßiger Reinheit, so daß bei der Aufarbeitung der Kondensate Isocyanat mit Alkohol zum Carbamat rekombiniert, das erneut in die Spaltung zurückgeführt werden muß. Dadurch leidet die Wirtschaftlichkeit des Verfahrens.

In der EP-PS 61 013 wird die Spaltung von Bis- und Polycarbamaten in Gegenwart von Lösungsmitteln und Hilfsmitteln, wie Chlorwasserstoff oder organischen Säurechloriden und gegebenenfalls Katalysatoren durchgeführt, wobei die Spaltprodukte wie in der EP-PS 54 817 mit Dephlegmatoren partiell kondensiert werden. Durch Verwendung von Lösungsmitteln und Hilfsmitteln, die unter den Reaktionsbedingungen flüchtig sind und zur Verunreinigung der Spaltprodukte führen, wird die Wirtschaftlichkeit noch weiter verschlechtert.

In der EP-PS 92 738 wird die Spaltung von Carbamaten in dünnem Film im Rohrreaktor bzw. Dünnschichtverdampfer durchgeführt, wo bei einmaligem Durchgang und kurzer Verweilzeit Folgereaktionen unterdrückt werden sollen. Da diese sich trotz Katalysator und/oder Stabilisator nicht restlos vermeiden lassen, werden Lösungsmittel eingesetzt, um Anbackungen im Rohrreaktor zu verhindern. Die gasförmigen Spaltprodukte werden mit hintereinandergeschalteten Dephlegmatoren partiell kondensiert. Die Spaltung zu Isophorondiisocyanat, die in Beispiel 4 beschrieben ist, zeigt die Nachteile des Verfahrens. So enthielt das am zweiten Dephlegmator abgezogene Roh-Isophorondiisocyanat nur 55,5 % Isophorondiisocyanat neben 43,2 % Monoisocyanato-monocarbamat und im anschließend kondensierten Roh-Butanol wurden noch 22,6 % Biscarbamat ausgewiesen. Die Ausbeute an Isophorondiisocyanat betrug lediglich 51,8 % d. Th.

In der EP-A 396 977 wird wie in der vorhergenannten EP-PS 92 738 die Spaltung von Carbamat in Gegenwart von Lösungsmittel und Katalysator in einem Rohrreaktor durchgeführt. Die gasförmigen Spaltprodukte werden ebenfalls durch partielle Kondensation in hintereinandergeschalteten Dephlegmatoren abgetrennt. Anschließend wird die Diisocyanatfraktion - nach weiterer Verdünnung mit dem zur Spaltung eingesetzten Lösungsmittel - mit Kohlenwasserstoffen extrahiert. Wegen der nur wenig unterschiedlichen Verteilungskoeffizienten vom Isophorondiisocyanat (IPDI) und Monoisocyanato-monocarbamat (IPIU) ist jedoch trotz mehrstufiger Extraktion nur eine unvollständige Trennung dieser Verbindungen möglich. Da außerdem mit dem Extraktionsmittel ein zusätzlicher Stoff in den Prozeß eingebracht wird, der in einem nachfolgenden Trennvorgang zurückgewonnen werden muß und dafür zusätzliche Investitionen erforderlich sind, ist das Verfahren noch unwirtschaftlicher geworden.

Die EP-A 396 976 unterscheidet sich von der EP-A 396 977 dadurch, daß die Spaltung entweder im Rohrreaktor oder diskontinuierlich im Rührreaktor durchgeführt wird, wobei die Temperatur und der Druck so gewählt werden, daß nur der Alkohol abdestilliert. Das Sumpfprodukt aus dem Rohrreaktor oder das Produkt aus dem Rührreaktor wird anschließend durch Extraktion und Destillation aufgearbeitet, so daß auch dies Verfahren ähnlich unwirtschaftlich ist.

Zusammenfassend ist festzuhalten, daß die Spaltung von Carbamaten in der Gasphase mit dem grundsätzlichen Nachteil der hohen thermischen Belastung der Reaktionsprodukte behaftet ist, während die Spaltung in der flüssigen Phase in Gegenwart von Lösungsmitteln einen höheren Energieaufwand und aufgrund der geringeren Raum/Zeit-Ausbeute entsprechend höhere Investitionen gegenüber der Spaltung in Flüssigphase ohne Lösungsmittel erfordert. Die zur partiellen Kondensation eingesetzten Dephlegmatoren arbeiten hinsichtlich einer zufriedenstellenden Trennung von Isocyanat und Alkohol wenig effektiv.

Der Erfindung liegt die Aufgabe zugrunde, ein verbessertes Verfahren zur Herstellung von (cyclo)aliphatischen Diisocyanaten bereitzustellen, das die oben genannten Nachteile vermeidet.

Gelöst wurde die Aufgabe durch das Finden eines kontinuierlichen, im wesentlichen aus drei Hauptschritten bestehenden Verfahrens, wobei im ersten Schritt unter speziellen Bedingungen die Bildung von Bisharnstoffen, im zweiten Schritt die Bildung von Biscarbamaten und im dritten Schritt die Spaltung der Biscarbamate in flüssiger Phase zu den Diisocyanaten erfolgt.

Gegenstand der Erfindung ist daher ein kontinuierliches mehrstufiges Verfahren zur Herstellung von (cyclo)aliphatischen Diisocyanaten der Formel

OCN - R¹ - NCO

wobei R¹ für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 6 bis 13 Kohlenstoffatomen steht, gekennzeichnet durch folgende Maßnahmen:
(a) Umsetzung von Harnstoff mit einem (cyclo)aliphatischen Diamin der Formel

   H₂N - R¹ - NH₂,

   in Gegenwart von Alkohol der Formel

   R² - OH

   als Lösungsmittel zu einem (Cyclo)alkylenbisharnstoff der Formel

   H₂N - CO - NH - R¹ - NH - CO - NH₂,

   wobei in den Formeln R¹ der oben genannten Definition entspricht und R² für einen Rest steht, wie er nach Entfernung der Hydroxylgruppe aus einem primären aliphatischen Alkohol mit 3 bis 5 Kohlenstoffatomen verbleibt, in einem Destillationsreaktor bei 100 bis 130 °C und einem Druck von 0,7 bis 1,5 bar (abs.), wobei die Edukte kontinuierlich auf den obersten Boden aufgegeben werden und das gebildete Ammoniak durch Destillation mit Alkoholbrüden, die im Sumpf eingeführt werden, ausgetrieben wird,
(b) Umsetzung des anfallenden Roh-(Cyclo)alkylenbisharnstoffs mit dem in (a) als Lösungsmittel eingesetzten Alkohol zum (Cyclo)alkylenbiscarbamat der Formel

   R² - O - CO - NH - R¹ - NH - CO - O - R²

   in einem Druckdestillationsreaktor, der bei Temperaturen von 160 bis 210 °C am Kopf und bei 180 bis 250 °C im Sumpf und einem Druck von 7 bis 13 bar betrieben wird, wobei das Reaktionsgemisch aus der ersten Reaktionsstufe (a) zusammen mit dem Reaktionsgemisch aus der Verfahrensstufe (e) auf den obersten Boden aufgegeben wird und wo durch Destillation mit Alkoholbrüden, die im Sumpf eingeführt werden, das gebildete Ammoniak ausgetrieben wird,
(c) Abtrennung von Ammoniak aus den am Kopf des Druckdestillationsreaktors (b) anfallenden Brüden und aus dem Alkohol, der durch partielle Kondensation der Brüden aus (a) gewonnen wird, in einer nachgeschalteten Kolonne zweckmäßigerweise unter dem Druck des Druckdestillationsreaktors, wobei der im Sumpf anfallende ammoniakfreie Alkohol in den Sumpf des Destillationsreaktors (a) und in den Sumpf des Druckdestillationsreaktors (b) zurückgeführt wird,
(d) Spaltung des gereinigten, mit Katalysator versehenen (Cyclo)alkylenbiscarbamats ohne Verwendung von Lösungsmitteln in einer kombinierten Spalt- und Rektifizierkolonne, in der im Unterteil unter Ausschleusung eines Teilstroms des Reaktionsgemisches gespalten wird und im Oberteil die Spaltprodukte rektifiziert werden, so daß am Kopf reiner Alkohol und im Seitenabzug Roh-(Cyclo)alkylendiisocyanat erhalten wird, das anschließend durch Vakuumdestillation gereinigt wird,
(e) Umsetzung der Ausschleusung aus dem Sumpf der kombinierten Spalt-und Rektifizierkolonne (d) mit dem Alkohol vom Kopf der kombinierten Spalt- und Rektifizierkolonne (d) und anschließende Rückführung des Reaktionsgemisches in den Druckdestillationsreaktor (b).

Im weiteren Text werden die Begriffe Diamin, Alkohol, Bisharnstoff, Biscarbamat und Diisocyanat im Sinne der o. g. Definitionen verwendet.

Überraschenderweise wurde gefunden, daß sich Harnstoff mit Diamin in Gegenwart von Alkohol als Lösungsmittel zu Bisharnstoff umsetzen läßt, wenn man die Reaktion bei Temperaturen von 100 bis 130 °C, d. h. unterhalb der Zersetzungstemperatur von Harnstoff, innerhalb von 4 bis 10 h unter Entfernung des gebildeten Ammoniaks durchführt. Dieser Bisharnstoff kann anschließend bei höheren Temperaturen von 160 bis 250 °C mit dem dann erst zur Reaktion gelangenden Alkohol nahezu quantitativ in Biscarbamat überführt werden.

Durch eine derartige Herstellung des Biscarbamats in zwei getrennten Stufen sowie unterschiedlichen Temperatur- und Druckbereichen gelingt es, Harnstoff bei relativ niedrigen Temperaturen selektiv mit dem Diamin zur Reaktion zu bringen und die Bildung von Nebenprodukten, wie sie aus den Patentschriften EP-PSS 18 586, EP 126 299, 126 300 sowie der EP-A 355 443 bekannt ist, zu unterdrücken.

Aufgrund der Ausführungen in der EP-PS 18 586 und JP-Sho 38-20748 war es nicht zu erwarten, daß die Umsetzung von Diaminen mit Harnstoff in Gegenwart von Alkohol bei Temperaturen unterhalb 130 °C selektiv zum Bisharnstoff erfolgt.

Durch Verwendung eines Destillations- und eines Druckdestillationsreaktors konnte eine kontinuierliche Herstellung des Biscarbamats erreicht und die Nachteile der Kaskaden- bzw. der alternierenden Fahrweise vermieden werden.

Darüber hinaus wurde gefunden, daß die Spaltung von (cyclo)aliphatischen Biscarbamaten in flüssiger Phase ohne Verwendung von Lösungsmitteln in einer kombinierten Spalt- und Rektifizierkolonne im technischen Maßstab ohne Probleme in hoher Ausbeute und hoher Reinheit vorgenommen werden kann, wenn diese mit hochwirksamen, vorzugsweise geordneten Packungen versehene Kolonne im Sumpf mit einem Fallfilmverdampfer ausgestattet ist, in dem maximal 20 Gew.-% der Aufgabe verdampft und Flüssigkeit und Brüden im Gleichstrom geführt werden, und wenn im oberen Drittel die Kolonne mit einer Einrichtung zum Abzug von Roh-Diisocyanat und am Kopf mit Kondensator, Kondensatsammelgefäß und Rückflußpumpe für die Abtrennung von reinem Alkohol versehen ist. Dadurch gelingt es, die Spaltung ohne Belegungen in der Spaltzone durchzuführen und ein relativ reines Roh-Diisocyanat und reinen Alkohol abzuziehen.

Zur Durchführung des erfindungsgemäßen Verfahrens zur kontinuierlichen Herstellung von (cyclo)aliphatischen Diisocyanaten werden (cyclo)aliphatische Diamine, Harnstoff und primäre aliphatische Alkohole eingesetzt.

Bei den (cyclo)aliphatischen Diaminen handelt es sich um organische Diamine mit aliphatisch und/oder cycloaliphatisch gebundenen Aminogruppen. Typische Beispiele sind aliphatische Diamine, wie z. B. Hexamethylendiamin, 2-Methylpentamethylendiamin, Octamethylendiamin, 2,2,4- und 2,4,4-Trimethylhexamethylendiamin oder Gemische davon, Decamethylendiamin, 2-Methylnonamethylendiamin, Dodecamethylendiamin und cycloaliphatische Diamine, wie z. B. 1,4-Cyclohexandiamin, 1,3- oder 1,4-Cyclohexandimethanamin, 5-Amino-1,3,3-trimethylcyclohexanmethanamin (Isophorondiamin), 2(4)-Methyl-1,3-cyclohexandiamin oder 4,4'-Methylenbis(cyclohexanamin).

Als Alkohole sind grundsätzlich alle primären aliphatischen Alkohole geeignet, die einerseits eine ausreichend große Differenz in der Siedetemperatur zum jeweiligen Diisocyanat aufweisen und andererseits ein Verdampfen des Biscarbamates und Kondensation der Spaltprodukte unter verfahrenstechnisch günstigen Betriebsdrücken gestatten. Infrage kommen daher Alkohole wie Propanol, Butanol, Isobutanol oder Pentanol.

Die Umsetzung von Diamin mit Harnstoff zum Bisharnstoff in Gegenwart von Alkohol als Lösungsmittel erfolgt in einem Destillationsreaktor, wobei die Edukte kontinuierlich auf den obersten Boden aufgegeben werden und das freigesetzte Ammoniak durch Alkoholbrüden, die im Sumpf des Destillationsreaktors eingeführt werden, ausgetrieben wird. Das Ammoniak/Alkohol-Gemisch wird, um die Abscheidung von Ammoniumcarbamat zu vermeiden, in einem Kondensator bei Temperaturen von 30 bis 50 °C partiell kondensiert. Aus dem Kondensat wird ammoniakfreier Alkohol durch Destillation in der dem Druckdestillationsreaktor nachgeschalteten Kolonne zurückgewonnen.

Das Molverhältnis der Edukte (cyclo)aliphatisches Diamin : Harnstoff : Alkohol beträgt 1 : 2,0 bis 2,4 : 3 bis 10. Der Destillationsreaktor besitzt mindestens 4 Böden. Die Reaktion wird bei Temperaturen von 100 bis 130 °C und Drücken von 0,7 bis 1,5 bar (abs.) durchgeführt. Die erforderliche Verweilzeit im Destillationsreaktor beträgt 4 bis 10 h, vorzugsweise 6 bis 8 h. Die zum Austreiben des Ammoniaks im Sumpf eingebrachte Alkoholmenge beträgt 0,05 bis 3 kg/kg, vorzugsweise 0,1 bis 1 kg/kg Bisharnstoff, wobei die so eingebrachte Alkoholmenge zusammen mit dem gebildeten Ammoniak am Kopf abgezogen, nach partieller Kondensation in einer Alkoholrückgewinnungskolonne von restlichen Ammoniak befreit und in den Sumpf zurückgeführt wird.

Um möglichst vollständige Umsetzung des Harnstoffs zum Bisharnstoff zu erreichen, ohne daß es bereits zur Bildung von (N-unsubstituiertem) O-Alkylcarbamat kommt, ist die Reaktionstemperatur auf maximal 130 °C begrenzt. Die aus der gewünschten Reaktionstemperatur, der Art und dem Verhältnis der Edukte sich ergebende Reaktionsgeschwindigkeit bestimmt die Verweilzeit und damit die Dimensionierung des Destillationsreaktors.

Der Vorteil des Destillationsreaktors gegenüber einer Rührkesselkaskade besteht darin, daß das Reaktionsgemisch in einer Destillationskolonne im Gegenstrom zu den im Sumpf eingebrachten Alkoholbrüden geführt wird, wobei jeder Boden praktisch einer Kaskadenstufe entspricht. Durch die eingebrachten Alkoholbrüden erfolgt eine derart intensive Durchmischung der Flüssigkeit auf den einzelnen Böden, daß entsprechende Rühreinrichtungen nicht mehr erforderlich sind. Daraus resultiert eine energetisch, betriebstechnisch und investitionsmäßig günstige Einrichtung. Der Energieaufwand ist erheblich geringer als bei der Rührkesselkaskade, da nur einmal die Alkoholbrüden erzeugt und kondensiert werden müssen. Entsprechend gering ist der apparative sowie meß- und regeltechnische Aufwand.

Der im Sumpf des Destillationsreaktors anfallende, in Alkohol gelöste Roh-Bisharnstoff wird kontinuierlich zusammen mit Kreislaufrückführung auf den obersten Boden des Druckdestillationsreaktors gefahren. Die Speisung kann außerhalb mit einem Wärmetauscher oder auch durch einen Einsteck-Erhitzer o. ä. in der Kolonne auf die erforderliche Reaktionstemperatur gebracht werden.

Hier erfolgt die Umsetzung des Bisharnstoffs mit dem Alkohol zum Biscarbamat bei erhöhter Temperatur und erhöhtem Druck, wobei Ammoniak freigesetzt wird, das aus Gleichgewichtsgründen aus dem Reaktionsgemisch entfernt werden muß. Dies geschieht durch Alkoholbrüden, die im Sumpf des Druckdestillationsreaktors eingeführt werden. Die Alkoholbrüden werden zweckmäßigerweise in einem am Sumpf der Kolonne angebrachten Verdampfer erzeugt.

Die Vorteile des Druckdestillationsreaktors gegenüber einer Rührkesselkaskade sind die gleichen wie beim Destillationsreaktor. Die für vollständigen Umsatz erforderlichen Stufen können bei Verwendung einer Rührkesselkaskade aus Kostengründen nicht realisiert werden, so daß dafür nicht vollständiger Umsatz und damit entsprechender Ausbeuteverlust in Kauf genommen werden muß (s. auch Ullmanns Encyklopädie der technischen Chemie, 4. Auflage 1973, Bd. 3, S. 342-349).

Die Reaktionsgeschwindigkeit der Biscarbamatbildung wird bestimmt durch die Parameter Temperatur, Druck, Verhältnis von Bisharnstoff zu Alkohol, eingebrachte Alkoholbrüden im Sumpf und Anzahl der Stufen des Druckdestillationsreaktors. Für das erfindungsgemäße Verfahren haben sich Drücke von 7 bis 13 bar, Temperaturen in Sumpf des Druckdestillationsreaktors von 180 bis 250 °C und am Kopf des Druckdestillationsreaktors von 160 bis 210 °C, ein molares Verhältnis von Bisharnstoff zu Alkohol 1 : 5 bis 12, eingebrachte Alkoholbrüden im Sumpf in einer Menge von 0,5 bis 8 kg/kg, vorzugsweise 1 bis 4 kg/kg gebildetem Biscarbamat als zweckmäßig erwiesen. Die zum vollständigen Umsatz erforderliche mittlere Verweilzeit im Druckdestillationsreaktor beträgt 5 bis 20 h, vorzugsweise 9 bis 14 h.

Aufgrund der geringen Reaktionsgeschwindigkeit der Umsetzung des Bisharnstoffs mit Alkohol ist eine hohe Temperatur wünschenswert, jedoch wegen der Bildung von Nebenprodukten auf maximal 250 °C begrenzt. Der Kolonnendruck stellt sich entsprechend ein und hängt dann nur noch vom verwendeten Alkohol und vom gewählten Gewichtsverhältnis Biscarbamat zu Alkohol im Sumpf ab. Dieses beträgt vorzugsweise 0,5 bis 1,7. Bei Verwendung von Butanol und einem Verhältnis von Biscarbamat zu Butanol im Sumpf des Druckdestillationsreaktors von 1:1 sowie einer Sumpftemperatur von 230 °C beträgt der Sumpfdruck etwa 11 bar. Dementsprechend stellt sich am Kopf der Kolonne eine Temperatur von ca. 200 °C ein.

Das am Kopf abgezogene dampfförmige Gemisch aus Alkohol und Ammoniak wird, ohne es zu kondensieren, vorzugsweise unter dem Druck des Druckdestillationsreaktors in den mittleren Bereich einer Destillationskolonne geführt, in der durch Rektifikation im Sumpf bei mindestens 170 °C, je nach gewähltem Alkohol und Betriebsdruck, ammoniakfreier Alkohol gewonnen wird, der in den Sumpf des Destillations- und des Druckdestillationsreaktors zurückgeführt wird. Am Kopf wird das Ammoniak flüssig abgezogen. Um die Belegung des Rückflußkondensators durch gegebenenfalls vorhandenes Ammoniumcarbamat zu verhindern, läßt man zur Temperaturerhöhung am Kopf auf mindestens 60 °C einen entsprechenden Anteil an Alkohol zu. Die so mit dem Ammoniak aus dem Kreislauf ausgetragene Alkoholmenge muß durch Frischalkohol ersetzt werden.

Das im Sumpf des Druckdestillationsreaktors anfallende Biscarbamat/Alkohol-Gemisch wird in an sich bekannter Weise destillativ gereinigt, wobei der abgetrennte Alkohol zweckmäßigerweise auf den obersten Boden des Destillationsreaktors zurückgeführt wird.

Dem gereinigten Biscarbamat wird vor Einsatz in die Spaltung der Katalysator als ca. 5 Gew.-%ige Lösung oder Suspension in dem Alkohol, der auch zur Herstellung des Biscarbamats verwendet wird, in einer Menge von 5 bis 400 ppm, vorzugsweise 20 bis 100 ppm, zudosiert. Als Katalysatoren eignen sich Halogenide oder Oxide von Metallen der Gruppen IB, IIB, IIIB, IVB, VB, VIB, VIIB und VIIIB des Periodensystems. Vorzugsweise werden Chloride von Zink oder Zinn sowie Oxide von Zink, Mangan, Eisen oder Cobalt verwendet.

Die Spaltung des Biscarbamats wird in einer kombinierten Spalt- und Rektifizierkolonne durchgeführt, in der im Unterteil gespalten wird und im Oberteil die Spaltprodukte rektifiziert werden. Das gebildete Diisocyanat wird als Roh-Diisocyanat im Seitenabzug gewonnen, während der reine Alkohol am Kopf abgezogen wird. Um bei der Spaltung gebildete Nebenprodukte zu entfernen, wird kontinuierlich aus dem Sumpf Reaktionsgemisch in einer Menge von 5 bis 50 Gew.-%, vorzugsweise 15 bis 25 Gew.-%, bezogen auf den Einsatz ausgeschleust. Die Spaltung wird bei einem Sumpfdruck von 5 bis 50 mbar, vorzugsweise 20 bis 30 mbar und einer Sumpftemperatur von 200 bis 260 °C, vorzugsweise 230 bis 240 °C durchgeführt. Die Einspeisung des zu spaltenden Biscarbamats kann alternativ in die Wälzung zum Fallfilmverdampfer oder in das untere Drittel der Kolonne, vorzugsweise oberhalb der Einrichtung zur Energierückgewinnung erfolgen.

Die kombinierte Spalt- und Rektifizierkolonne ist für die Energiezufuhr im Sumpf mit einem Fallfilmverdampfer, im unteren Drittel mit einer Einrichtung zur Energierückgewinnung, im oberen Drittel mit einer Einrichtung zum Abzug von Roh-Diisocyanat und am Kopf mit einem Kondensator, Kondensatsammelgefäß und Pumpe für den Rückfluß und den Abzug von reinem Alkohol ausgestattet.

Um eine zu hohe thermische Belastung des Biscarbamats zu vermeiden, wird der Fallfilmverdampfer für die Energiezufuhr im Sumpf der Kolonne so betrieben, daß bei einmaligem Durchgang maximal 20 Gew.-%, vorzugsweise weniger als 10 Gew.-% der Aufgabe verdampfen und daß Flüssigkeit und Brüden im Gleichstrom geführt werden.

Aufgrund der Reaktivität der Isocyanatgruppen sollte deren mittlere Verweilzeit in der Spaltzone möglichst gering sein, was durch Minimierung des Flüssigkeitsvolumens durch entsprechende konstruktive Maßnahmen und durch Verwendung von geordneten Packungen mit geringem 'hold up' sowie durch möglichst unverzügliche destillative Entfernung des gebildeten Diisocyanats aus der Spaltzone erreicht wird. Letzteres wird durch entsprechenden Energieeintrag im Sumpf der kombinierten Spalt- und Rektifizierkolonne realisiert. Dadurch stellt sich ein Konzentrationsprofil in der Kolonne ein, wo im Sumpf im wesentlichen Biscarbamat, weniger als 3 Gew.-% Diisocyanat und nicht nachweisbare Mengen an Alkohol vorhanden sind, während die Flüssigkeit im unteren Teil der Kolonne nur noch geringe Mengen an Biscarbamat, dagegen in wesentlichen aus Monoisocyanatomonocarb-amat besteht. Der dazu erforderliche Rücklauf wird zweckmäßigerweise durch eine Kondensationstufe oberhalb der Spaltzone und unterhalb der des Diisocyanatseitenabzugs erzeugt. Diese Betriebsweise ist besonders wirtschaftlich, da die hier abzuführende Energie auf relativ hohem Temperaturniveau anfällt und anschließend nochmals genutzt werden kann z. B. zur Aufwärmung der Einsatzprodukte, die in den Destillationsreaktor zur Bisharnstoff-Herstellung geführt werden. Außerdem wird dadurch die Brüdenmenge entsprechend verringert, so daß oberhalb dieses Partialkondensators der Durchmesser der Kolonne entsprechend reduziert werden kann.

Trotz der möglichst unverzüglichen destillativen Entfernung des gebildeten Diisocyanats aus der Spaltzone läßt sich die Bildung von höhermolekularen Verbindungen nicht restlos verhindern, so daß kontinuierlich ein dementsprechender Anteil aus dem Sumpf der kombinierten Spalt- und Rektifizierkolonne ausgeschleust werden muß. Diese Produkte werden zur Umsetzung mit dem Alkohol vom Kopf der kombinierten Spalt- und Rektifizierkolonne in einen nachgeschalteten Reaktor geführt.

Die Reinigung des aus der kombinierten Spalt- und Rektifizierkolonne abgezogenen Roh-Diisocyanats erfolgt in an sich bekannter Weise durch Vakuumdestillation. Vorlauf und Destillationsrückstand werden zweckmäßigerweise in die kombinierte Spalt- und Rektifizierkolonne zurückgeführt. Die Ausschleusung aus dem Sumpf der kombinierten Spalt- und Rektifizierkolonne sowie der dort am Kopf abgezogene Alkohol werden kontinuierlich gemischt und nach Erwärmung auf 100 bis 140 °C in einem Rohrreaktor unter einem Druck von 2 bar und einer Verweilzeit von 1 bis 4 h, vorzugsweise 2 h umgesetzt, um die Isocyanatgruppen in Carbamatgruppen zu überführen. Das Reaktionsprodukt wird kontinuierlich in den Druckdestillationsreaktor auf den obersten Boden zurückgeführt.

### Beschreibung der Anlage

Im folgenden soll die Einrichtung, in der das erfindungsgemäße Verfahren zur kontinuierlichen mehrstufigen Herstellung von (cyclo)aliphatischen Diisocyanaten gemäß Beispiel 1 bis 4 durchgeführt wurde, anhand der Fit. 1 beschrieben werden. Die entsprechenden Stoffströme sind in Klammern angeführt.

Aus dem Mischgefäß A wird kontinuierlich mit einer Pumpe ein Gemisch (4) von Diamin (1), Harnstoff (2) und Alkohol (3) über den mit Dampf beheizten Vorwärmer auf den obersten Boden des Destillationsreaktors B gefahren. Die mittlere Verweilzeit im Destillationsreaktor B beträgt 7 h. Im Sumpf des unter Normaldruck betriebenen Destillationsreaktors B wird Alkohol (5) aus dem Sumpf der Kolonne D eingeführt. Die im Reboiler dem Destillationsreaktor B zugeführte Energiemenge wird so eingeregelt, daß die am Kopf zusammen mit dem gebildeten Ammoniak (6) anfallende und im Dephlegmator mit Warmwasser von 40 °C kondensierte Alkoholmenge der im Sumpf eingebrachten entspricht. Der so kondensierte Alkohol wird kontinuierlich in die Kolonne D gefahren.

Die im Sumpf des Destillationsreaktors B anfallende Lösung von Bisharnstoff in Alkohol wird standgeregelt über einen Vorwärmer, wo sie auf 190 bis 200 °C aufgeheizt wird, zusammen mit dem Reaktionsprodukt (18) aus dem Reaktor K auf den obersten Boden des Druckdestillationsreaktors C gefahren. Die mittlere Verweilzeit im Druckdestillationsreaktor C beträgt 10,5 h. Durch Heizung wird folgendes Temperaturprofil eingestellt: Sumpf 229 ° und Kopf 200 °C. Im Sumpf des Druckdestillationsreaktors C wird Alkohol (7) eingeführt und die Wärmeträgerölmenge zum Reboiler so eingeregelt, daß die am Kopf zusammen mit dem gebildeten Ammoniak abgezogene Alkoholmenge der im Sumpf eingeführten entspricht.

Das erhaltene Alkohol/Ammoniak-Gemisch wird anschließend in die Alkoholrückgewinnungskolonne D gefahren. Die Kopftemperatur beträgt 85 °C. Die Alkoholverluste, die durch die Ammoniakausschleusung (6 und 9) entstehen, werden durch standgeregelte Zufuhr von Frischalkohol (10) in dem Sumpf der Kolonne D ersetzt. Das im Sumpf des Druckdestillationsreaktors C anfallende Gemisch (8) wird in an sich bekannter Weise durch Destillation (E) gereinigt.

Die für die Spaltung des Biscarbamats verwendete kombinierte Spalt- und Rektifizierkolonne F ist wie folgt aufgebaut: Im Unterteil befindet sich geordnete Packung, oberhalb dieser ein Röhrenbündelwärmetauscher. Darüber ist die Kolonne auf 50 % des unteren Querschnittes eingezogen und mit geordneter Packung mit zwischenliegender Flüssigkeitsverteilung und schließlich mit Brüdenrohr zum Kopfkondensator und Kondensatsammelgefäß G ausgestattet.

Die Einspeisung des zu spaltenden Biscarbamats (11) erfolgt nach Zusatz von Katalysator (12) auf dem oberen Boden des Röhrenbündelwärmetauschers, dessen Rohre 10 mm über den Boden herausragen, um eine gleichmäßige Verteilung über den gesamten Querschnitt zu erzielen. Die für die Spaltung und Rektifikation erforderliche Energie wird mit Wärmeträgeröl im Fallfilmverdampfer H übertragen. Das den Fallfilmverdampfer verlassende Dampf-Flüssigkeitsgemisch (13) trennt sich im Sumpf der kombinierten Spalt- und Rektifizierkolonne F.

Der während der Spaltung gebildete, durch Rektifikation am Kopf anfallende reine Alkohol wird aus dem Kondensatsammelgefäß G abgezogen (15) und mit der Sumpfausschleusung (17) in den Reaktor K geführt.

Aus dem Roh-Diisocyanatabzug (14) wird in an sich bekannter Weise durch Vakuumdestillation (J) das gereinigte Diisocyanat (16) erhalten.

Das nach Umsetzung von Isocyanatgruppen mit Alkohol bei 120 °C und einer Verweilzeit von ca. 2 h im Rohrreaktor K erhaltene Produktgemisch (18) wird anschließend auf den obersten Boden des Druckdestillationsreaktors C gegeben.

### Beispiel 1 bis 4

**Tabelle 1**

| Stoffstrom | | | Beispiel | | | |
|---|---|---|---|---|---|---|
| Nr. | Bezeichnung | Dimension | 1 | 2 | 3 | 4 |
| 1 | Diamin | kg/h | IPD 23,8 | TMD 20,5 | MPD 14,3 | IPD 18,4 |
| 2 | Harnstoff | kg/h | 17,3 | 16,0 | 15,2 | 13,4 |
| 3 | Butanol | kg/h | 62,2 | 57,6 | 54,7 | 48,1 |
| 4 | Edukte | °C | 119 | 119 | 118 | 119 |
| 5 | Butanol | kg/h | 6,9 | 6,4 | 6,1 | 6,2 |
| 6 | Butanol | Vol.-% | 2,5 | 2,5 | 2,5 | 2,5 |
| 7 | Butanol | kg/h | 56,7 | 52,9 | 51,0 | 51,8 |
| 8 | Biscarbamat:Butanol | kg/kg | 1,16 | 1,22 | 1,12 | 1,30 |
| 9 | Butanol | Gew.-% | 11,1 | 11,1 | 10,0 | 11,0 |
| 10 | Butanol | kg/h | 2,7 | 2,7 | 2,8 | 2,6 |
| 11 | Biscarbamat | kg/h | 65,6 | 63,0 | 54,6 | 55,1 |
| | n_{D}²⁵ | - | 1,4778 | 1,4654 | - | 1,4778 |
| | Schmelzpunkt | °C | - | - | 48 | - |
| 12 | 5 % ZnCl₂ in Butanol | kg/h | 0,13 | 0,12 | 0,11 | - |
| | 5 % ZnO in Butanol | kg/h | - | - | - | 0,11 |
| 13 | Verdampfung | Gew.-% | 9,0 | 8,5 | 8,0 | 9,0 |
| | Druck | mbar | 27 | 27 | 27 | 27 |
| 14 | Roh-Diisocyanat | kg/h | 32,0 | 28,0 | 21,8 | 24,7 |
| | Butanol | Gew.-% | 0,53 | 0,62 | 0,95 | 0,85 |
| 15 | Butanol | kg/h | 23,1 | 22,1 | 21,3 | 18,4 |
| 16 | Rein-Diisocyanat | kg/h | 30,0 | 26,0 | 19,6 | 22,5 |
| | Ausbeute | % | 96,5 | 95,4 | 94,6 | 93,6 |
| | n_{D}²⁵ | - | 1,4832 | 1,4614 | 1,4537 | 1,4832 |
| 17 | Ausschleusung | kg/h | 12,5 | 14,8 | 13,6 | 14,1 |
| 18 | Rückführung | kg/h | 35,8 | 37,1 | 35,1 | 32,7 |
| | NCO-Gehalt | Gew.-% | <0,1 | <0,1 | <0,1 | <0,1 |
| IPD = 5-Amino-1,3,3-trimethylcyclohexanmethanamin TMD = 2,2,4(2,4,4)-Trimethylhexamethylendiamin (Gemisch) MPD = 2-Methylpentamethylendiamin | | | | | | |

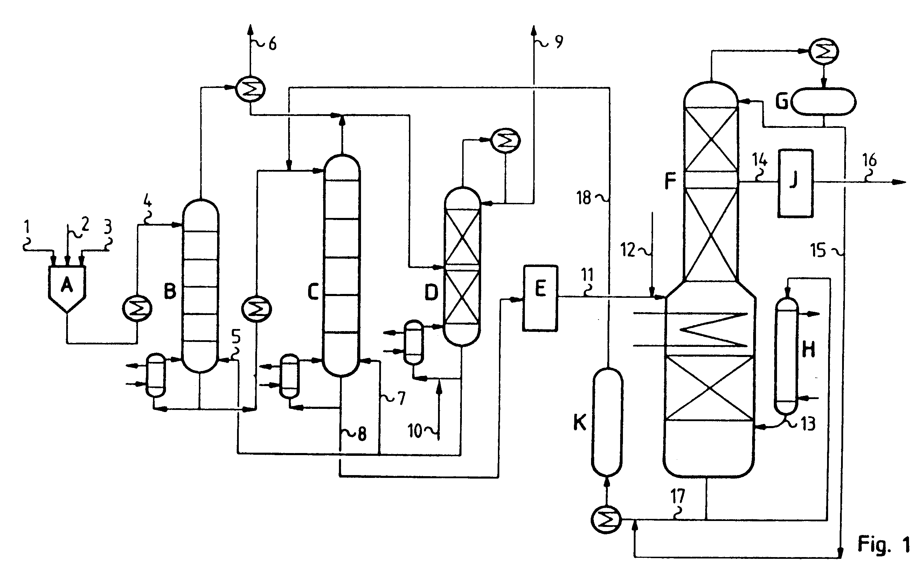

## Patentansprüche

1. Kontinuierliches mehrstufiges Verfahren zur Herstellung von (cyclo)aliphatischen Diisocyanaten der Formel
OCN - R¹ - NCO
wobei R¹ für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 6 bis 13 Kohlenstoffatomen steht, gekennzeichnet durch folgende Maßnahmen:
(a) Umsetzung von Harnstoff mit einem (cyclo)aliphatischen Diamin der Formel
H₂N - R¹ - NH₂,
in Gegenwart von Alkohol der Formel
R² - OH
als Lösungsmittel zu einem (Cyclo)alkylenbisharnstoff der Formel
H₂N - CO - NH - R¹ - NH - CO - NH₂,
wobei in den Formeln R¹ der oben genannten Definition entspricht und R² für einen Rest steht, wie er nach Entfernung der Hydroxylgruppe aus einem primären aliphatischen Alkohol mit 3 bis 5 Kohlenstoffatomen verbleibt, in einem Destillationsreaktor mit mindestens 4 Böden bei 100 bis 130 °C und einem Druck von 0,7 bis 1,5 bar (abs.), wobei die Edukte kontinuierlich auf den obersten Boden aufgegeben werden und das gebildete Ammoniak durch Destillation mit Alkoholbrüden, die im Sumpf eingeführt werden, ausgetrieben wird,
(b) Umsetzung des anfallenden Roh-(Cyclo)alkylenbisharnstoffs mit dem in (a) als Lösungsmittel eingesetzten Alkohol zum (Cyclo)alkylenbiscarbamat der Formel
R² - O - CO - NH - R¹ - NH - CO - O - R²
in einem Druckdestillationsreaktor, der am Kopf bei Temperaturen von 160 bis 210 °C und im Sumpf bei 180 bis 250 °C und einem Druck von 7 bis 13 bar betrieben wird, wobei das Reaktionsgemisch aus der ersten Reaktionsstufe (a) zusammen mit dem Reaktionsgemisch aus der Verfahrensstufe (e) auf den obersten Boden aufgegeben wird und wo durch Destillation mit Alkoholbrüden, die im Sumpf eingeführt werden, das gebildete Ammoniak ausgetrieben wird,
(c) Abtrennung von Ammoniak aus den am Kopf des Druckdestillationsreaktors (b) anfallenden Brüden und aus dem Alkohol, der durch partielle Kondensation der Brüden aus (a) gewonnen wird, in einer nachgeschalteten Kolonne zweckmäßigerweise unter dem Druck des Druckdestillationsreaktors, wobei der im Sumpf anfallende ammoniakfreie Alkohol in den Sumpf des Destillationsreaktors (a) und in den Sumpf des Druckdestillationsreaktors (b) zurückgeführt wird,
(d) Spaltung des gereinigten, mit Katalysator versehenen (Cyclo)alkylenbiscarbamats ohne Verwendung von Lösungsmitteln in einer kombinierten Spalt- und Rektifizierkolonne, in der im Unterteil unter Ausschleusung eines Teilstroms des Reaktionsgemisches gespalten wird und im Oberteil die Spaltprodukte rektifiziert werden, so daß am Kopf reiner Alkohol und im Seitenabzug Roh-(Cyclo)alkylendiisocyanat erhalten wird, das anschließend durch Vakuumdestillation gereinigt wird,
(e) Umsetzung der Ausschleusung aus dem Sumpf der kombinierten Spalt- und Rektifizierkolonne (d) mit dem Alkohol vom Kopf der kombinierten Spalt- und Rektifizierkolonne (d) und anschließende Rückführung des Reaktionsgemisches in den Druckdestillationsreaktor (b).

2. Verfahren gemäß Anspruch 1,
dadurch gekennzeichnet,
daß (cyclo)aliphatisches Diamin, Harnstoff und Alkohol in der Reaktionsstufe (a) im Molverhältnis 1 : 2,0 bis 2,4 : 3 bis 10 eingesetzt werden.

3. Verfahren gemäß den Ansprüchen 1 und 2,
dadurch gekennzeichnet,
daß im Sumpf des Destillationsreaktors die eingebrachte Alkoholmenge 0,05 bis 3 kg/kg (Cyclo)alkylenbisharnstoff, vorzugsweise 0,1 bis 1 kg/kg (Cyclo)alkylenbisharnstoff beträgt und daß die so eingebrachte Alkoholmenge dampfförmig am Kopf zusammen mit dem Ammoniak abgezogen, nach partieller Kondensation in der Alkohol-Rückgewinnungskolonne (c) vom restlichen Ammoniak befreit und in den Sumpf zurückgeführt wird.

4. Verfahren gemäß den Ansprüchen 1 bis 3,
dadurch gekennzeichnet,
daß die Verweilzeit im Destillationsreaktor 4 bis 10 h, vorzugsweise 6 bis 8 h beträgt.

5. Verfahren gemäß Anspruch 1,
dadurch gekennzeichnet,
daß die im Sumpf des Druckdestillationsreaktors eingebrachte Alkoholmenge 0,5 bis 8 kg/kg gebildetem (Cyclo)alkylenbiscarbamat, vorzugsweise 1 bis 4 kg/kg gebildetem (Cyclo)alkylenbiscarbamat beträgt und daµ sie vollständig verdampft und in Dampfform am Kopf des Druckdestillationsreaktors abgezogen wird.

6. Verfahren gemäß den Ansprüchen 1 und 5,
dadurch gekennzeichnet,
daß die Energiezufuhr im Sumpf des Druckdestillationsreaktors so eingeregelt wird, daß dort das Gewichtsverhältnis (Cyclo)alkylenbiscarbamat : Alkohol 0,5 bis 1,7 beträgt.

7. Verfahren gemäß den Ansprüchen 1, 5 und 6,
dadurch gekennzeichnet,
daß die Verweilzeit im Druckdestillationsreaktor 5 bis 20 h, vorzugsweise 9 bis 14 h beträgt.

8. Verfahren gemäß den Ansprüchen 1, 5 und 6,
dadurch gekennzeichnet,
daß die am Kopf des Druckdestillationsreaktors anfallenden Brüden in der nachgeschalteten Kolonne unter Systemdruck und einer Kopftemperatur von mindestens 60 °C und einer Sumpftemperatur je nach gewähltem Alkohol und Betriebsdruck von mindestens 170 °C vom Ammoniak befreit werden.

9. Verfahren gemäß Anspruch 1,
dadurch gekennzeichnet,
daß als Katalysatoren für die Spaltung des (Cyclo)alkylenbiscarbamats Halogenide oder Oxide von Metallen der Gruppen IB, IIB, IIIB, IVB, VB, VIB, VIIB und VIIIB des Periodensystems, vorzugsweise Chloride von Zink oder Zinn oder Oxide von Zink, Mangan, Eisen oder Cobalt, verwendet werden und daß die Katalysatorkonzentration, bezogen auf eingesetztes (Cyclo)alkylenbiscarbamat 5 bis 400 ppm, vorzugsweise 20 bis 200 ppm, beträgt.

10. Verfahren gemäß Anspruch 1,
dadurch gekennzeichnet,
daß die kombinierte Spalt- und Rektifizierkolonne, die mit minimalem Flüssigkeitsvolumen im Sumpf betrieben wird, für die Energiezufuhr im Sumpf mit einem Fallfilmverdampfer, im unteren Drittel mit einer Einrichtung zur Energierückgewinnung, im oberen Drittel mit einer Einrichtung zum Abzug von Roh-(Cyclo)alkylendiisocyanat und am Kopf mit Kondensator, Kondensatsammelgefäß und Pumpe für den Rückfluß und den Abzug von reinem Alkohol versehen ist.

11. Verfahren gemäß den Ansprüchen 1 und 10,
dadurch gekennzeichnet,
daß im Fallfilmverdampfer maximal 20 %, vorzugsweise weniger als 10 % der Produktaufgabe verdampft und daß Flüssigkeit und Brüden im Gleichstrom geführt werden.

12. Verfahren gemäß Anspruch 1,
dadurch gekennzeichnet,
daß kontinuierlich aus dem Sumpf der kombinierten Spalt- und Rektifizierkolonne, bezogen auf Einsatz 5 bis 50 %, vorzugsweise 15 bis 25 % ausgeschleust werden.

13. Verfahren gemäß Anspruch 1,
dadurch gekennzeichnet,
daß der Reaktor, in dem die Produkte aus der Spaltung - Alkohol vom Kopf und Ausschleusung vom Sumpf der kombinierten Spalt- und Rektifizierkolonne (d) - kontinuierlich umgesetzt werden, als Rohrreaktor ausgebildet ist und bei einer Temperatur von 100 bis 140 °C, einem Druck von 2 bar und einer Verweilzeit von 1 bis 4 h, vorzugsweise 2 h betrieben wird.

## Claims

1. Continuous multi-stage process for the production of (cyclo)aliphatic dilsocyanates of the formula
OCN - R¹ - NCO,
R¹ standing for a straight-chained or branched aliphatic hydrocarbon radical with a total of 6 to 12 carbon atoms or for an optionally substituted cycloaliphatic hydrocarbon radical with a total of 6 to 13 carbon atoms, characterized by the following measures:
(a) reaction of urea with a (cyclo)aliphatic diamine of the formula
H₂N - R¹ - NH₂
in the presence of alcohol of the formula
R² - OH
as solvent to give a (cyclo)alkylene bisurea of the formula
H₂N - CO - NH - R¹- NH - CO - NH₂,
R¹ corresponding to the definition given above in the formulae and R² standing for a radical such as remains following removal of the hydroxyl group from a primary aliphatic alcohol with 3 to 5 carbon atoms, in a distillation reactor with at least 4 trays at 100 to 130 °C and at a pressure of 0.7 to 1.5 bar (abs.), the educts being continuously loaded onto the topmost tray and the formed ammonia being expelled by distillation with alcohol vapours which are introduced in the base,
(b) reaction of the resulting untreated (cyclo)alkylene bisurea with the alcohol used in (a) as solvent to give the (cyclo)alkylene biscarbamate of the formula
R² - O - CO - NH - R¹ - NH - CO - O - R²
in a pressure distillation reactor which is operated at the head at temperatures of 160 to 210 °C and in the base at 180 to 250 °C and at a pressure of 7 to 13 bar, in which the reaction mixture from the first reaction stage (a) is loaded together with the reaction mixture from process stage (e) onto the topmost tray and where the formed ammonia is expelled by distillation with alcohol vapours which are introduced in the base,
(c) separation of ammonia from the vapours resulting at the head of the pressure distillation reactor (b) and from the alcohol which is obtained by partial condensation of the vapours from (a), in a downstream column expediently below the pressure of the pressure distillation reactor, the ammonia-free alcohol resulting in the base being recycled into the base of the distillation reactor (a) and into the base of the pressure distillation reactor (b),
(d) splitting of the purified (cyclo)alkylene biscarbamate, provided with catalyst, without using solvents in a combined splitting and rectifying column, in which splitting is carried out in the lower part accompanied by flushing out of a part-stream of the reaction mixture and the splitting products are rectified in the upper part, so that pure alcohol is obtained at the head and untreated (cyclo)alkylene diisocyanate is obtained in the side offtake and subsequently purified by vacuum distillation,
(e) reaction of the material flushed out from the base of the combined splitting and rectifying column (d) with the alcohol from the head of the combined splitting and rectifying column (d) and subsequent recycling of the reaction mixture into the pressure distillation reactor (b).

2. Process according to claim 1,
characterized in that
(cyclo)aliphatic diamine, urea and alcohol are used in the reaction stage (a) in the molar ratio 1 : 2.0 to 2.4 : 3 to 10.

3. Process according to claims 1 and 2,
characterized in that
in the base of the distillation reactor the introduced quantity of alcohol is 0.05 to 3 kg/kg (cyclo)alkylene bisurea, preferably 0.1 to 1 kg/kg (cyclo)alkylene bisurea and in that the thus-introduced quantity of alcohol is drawn off in vapour form at the head together with the ammonia, freed of the remaining ammonia after partial condensation in the alcohol-recovery column (c) and recycled into the base.

4. Process according to claims 1 to 3,
characterized in that
the residence time in the distillation reactor is 4 to 10 h, preferably 6 to 8 h.

5. Process according to claim 1,
characterized in that
the quantity of alcohol introduced in the base of the pressure distillation reactor is 0.5 to 8 kg/kg formed (cyclo)alkylene biscarbamate, preferably 1 to 4 kg/kg formed (cyclo)alkylene biscarbamate, and in that it is completely evaporated and drawn off in vapour form at the head of the pressure distillation reactor.

6. Process according to claims 1 and 5,
characterized in that
the energy supply in the base of the pressure distillation reactor is regulated such that the (cyclo)alkylene biscarbamate : alcohol weight ratio there is 0.5 to 1.7.

7. Process according to claims 1, 5 and 6,
characterized in that
the residence time in the pressure distillation reactor is 5 to 20 h, preferably 9 to 14 h.

8. Process according to claims 1, 5 and 6
characterized in that
the vapours occurring at the head of the pressure distillation reactor are freed of the ammonia in the downstream column under system pressure and at a head temperature of at least 60 °C and a base temperature, depending on the chosen alcohol and operating pressure, of at least 170 °C.

9. Process according to claim 1,
characterized in that
halides or oxides of metals of groups IB, IIB, IIIB, IVB, VB, VIB, VIIB and VIIIB of the periodic system, preferably chlorides of zinc or tin or oxides of zinc, manganese, iron or cobalt, are used as catalysts for the splitting of the (cyclo)alkylene biscarbamate and in that the catalyst concentration, relative to (cyclo)alkylene biscarbamate used, is 5 to 400 ppm, preferably 20 to 200 ppm.

10. Process according to claim 1,
characterized in that
the combined splitting and rectifying column, which is operated with a minimal liquid volume in the base, is provided with a falling-film evaporator in the base for the energy supply, with an energy-recovery device in the lower third, with a device for drawing off untreated (cyclo)alkylene diisocyanate in the upper third and with a condenser, condensate collection vessel and pump for the return flow and the drawing-off of pure alcohol at the head.

11. Process according to claims 1 and 10,
characterized in that
a maximum of 20 %, preferably less than 10% of the product load is evaporated in the falling-film evaporator and in that liquid and vapours are guided in parallel flow.

12. Process according to claim 1,
characterized in that
relative to the feed, 5 to 50%, preferably 15 to 25% are flushed out continuously from the base of the combined splitting and rectifying column.

13. Process according to claim 1,
characterized in that
the reactor in which the products from the splitting - alcohol from the head and flushed-out material from the base of the combined splitting and rectifying column (d) - are continuously reacted is designed as a tubular reactor and is operated at a temperature of 100 to 140 °C, a pressure of 2 bar and with a residence time of 1 to 4 h, preferably 2 h.

## Revendications

1. Procédé en plusieurs étapes en continu pour la production de diisocyanates (cyclo)aliphatiques de formule :
OCN - R¹ - NCO
dans laquelle R¹ représente un radical hydrocarboné aliphatique à chaîne droite ou ramifié ayant au total 6 à 12 atomes de carbone ou un radical hydrocarboné cycloaliphatique éventuellement substitué ayant au total de 6 à 13 atomes de carbone,
caractérisé par les mesures suivantes :
a) réaction de l'urée avec une diamine (cyclo)aliphatique de formule :
H₂N - R¹ - NH₂,
en présence d'un alcool de formule :
R² - OH
comme solvant, en une (cyclo)alcoylène bis-urée de formule :
H₂N - CO - NH - R¹ - NH - CO - NH₂,
dans laquelle, dans les formules, R¹ correspond à la définition citée ci-dessus et R² représente un radical comme il reste après élimination du groupe hydroxyle d'un alcool primaire aliphatique ayant de 3 à 5 atomes de carbone, dans un réacteur de distillation de 100 à 130°C et à une pression de 0,7 à 1,5 bar (abs.), dans lequel les éducts sont chargés en continu sur le plateau le plus élevé et l'ammoniac formé est chassé par distillation avec des vapeurs d'alcool qui ont été introduites dans le pied de colonne,
b) réaction de la (cyclo)alcoylène bis-urée brute qui s'est produite, avec l'alcool mis en jeu en a) comme solvant, en (cyclo)alcoylène bis-carbamate de formule :
R² - O - CO - NH - R¹ - NH - CO - O - R²
dans un réacteur de distillation sous pression qui est mis en fonctionnement à des températures de 160 à 210°C en tête et de 180 à 250°C en pied de colonne et à une pression de 7 à 13 bars, dans lequel le mélange réactionnel provenant de la première étape de réaction a) est chargé ensemble avec le mélange réactionnel provenant de l'étape de procédé e) sur le plateau le plus élevé et où par distillation avec des vapeurs d'alcool qui ont été introduites en pied de colonne et l'ammoniac formé est chassé.
c) séparation de l'ammoniac des vapeurs produites en tête du réacteur de distillation sous pression b) et de l'alcool qui est obtenu par condensation partielle des vapeurs provenant de a) dans une colonne post-connectée, d'une manière appropriée, sous la pression du réacteur de distillation sous pression, dans lequel l'alcool dépourvu d'ammoniac qui se produit en pied de colonne, est recyclé dans le pied de colonne du réacteur de distillation a) et dans le pied de colonne du réacteur de distillation sous pression b),
d) division du (cyclo)alcoylène bis-carbamate purifié pourvu du catalyseur, sans utilisation de solvants dans une colonne combinée de division et de rectification dans laquelle à la base tout en évacuant un courant partiel du mélange réactionnel, on effectue la division et à la partie supérieure on rectifie le produit de division, de sorte qu'en tête on obtient l'alcool pur et par une décharge latérale le (cyclo)alcoylènediisocyanate brut, qui est purifié ensuite par distillation sous vide,
e) réaction de l'évacuation provenant du pied de colonne de la colonne combinée de division et de rectification (d) avec l'alcool provenant de la tête de la colonne combinée de division et de rectification (d) et retour consécutif du mélange réactionnel dans le réacteur de distillation sous pression b).

2. Procédé selon la revendication 1, caractérisé en ce que la diamine (cyclo)aliphatique, l'urée et l'alcool sont mis en oeuvre dans le stade de réaction a) dans le rapport molaire 1 : 2,0 à 2,4 : 3 à 10.

3. Procédé selon les revendications 1 et 2, caractérisé en ce que dans le pied de colonne du réacteur de distillation, la quantité d'alcool introduite s'élève de 0,05 à 3 kg/kg de (cyclo)alcoylène bis-urée, de préférence de 0,1 à 1 kg/kg de (cyclo)alcoylène bis-urée et en ce que la quantité d'alcool ainsi introduite est soutirée sous forme de vapeur, en tête, conjointement avec l'ammoniac, est débarrassé après condensation partielle dans la colonne de récupération de l'alcool (C), de l'ammoniac résiduel et est ramené dans le pied de colonne.

4. Procédé selon les revendications 1 à 3, caractérisé en ce que le temps de séjour dans le réacteur de distillation s'élève de 4 à 10 heures, de préférence de 6 à 8 heures.

5. Procédé selon la revendication 1, caractérisé en ce que la quantité d'alcool introduite dans le pied de colonne du réacteur de distillation sous pression s'élève de 0,5 à 8 kg/kg de (cyclo)alcoylène bis-carbamate formé, de préférence de 1 à 4 kg/kg de (cyclo)alcoylène bis-carbamate formé et en ce qu'elle s'évapore complètement et est soutirée sous forme de vapeur en tête du réacteur de distillation sous pression.

6. Procédé selon les revendications 1 et 5, caractérisé en ce que l'amenée d'énergie dans le pied de colonne du réacteur de distillation sous pression est régulée de telle sorte que là le rapport en poids (cyclo)alcoylène bis-carbamate/alcool s'élève de 0,5 à 1,7.

7. Procédé selon les revendications 1, 5 et 6, caractérisé en ce que le temps de séjour dans le réacteur de distillation sous pression, s'élève de 5 à 20 heures, de préférence de 9 à 14 heures.

8. Procédé selon les revendications 1, 5 et 6, caractérisé en ce que les vapeurs qui se produisent en tête du réacteur de distillation sous pression, sont débarrassées de l'ammoniac dans la colonne post-connectée sous pression d'ensemble et à une température de tête d'au moins 60°C, et à une température de pied de colonne selon l'alcool choisi et la pression de fonctionnement, d'au moins 170°C.

9. Procédé selon la revendication 1, caractérisé en ce que comme catalyseurs pour la division du (cyclo)alcoylène bis-carbamate, on utilise des halogénures ou des oxydes de métaux des groupes IB, IIB, IIIB, IVB, VB, VIB, VIIB et VIIIB du système périodique, de préférence le chlorure de zinc ou d'étain, ou les oxydes de zinc, de manganèse, de fer ou de cobalt, et en ce que la concentration du catalyseur, rapporté au (cyclo)alcoylène bis-carbamate mis en oeuvre s'élève de 5 à 400 ppm, de préférence de 20 à 200 ppm.

10. Procédé selon la revendication 1, caractérisé en ce que la colonne combinée de division et de rectification qui est mise en fonctionnement avec le volume minimal de liquide dans le pied de colonne, est pourvue pour l'apport d'énergie dans le pied de colonne, d'un évaporateur à film tombant, dans le tiers inférieur d'un dispositif pour la récupération de l'énergie, dans le tiers supérieur d'un dispositif pour la décharge du (cyclo)alcoylènediisocyanate brut et en tête d'un condenseur, d'un récipient de recueil du condensat et d'une pompe pour le reflux et le soutirage de l'alcool pur.

11. Procédé selon les revendications 1 et 10, caractérisé en ce que dans l'évaporateur à film tombant au maximum 20 %, de préférence moins de 10 % de la charge en produit est évaporé et en ce que le liquide et les vapeurs sont conduits en courants parallèles.

12. Procédé selon la revendication 1, caractérisé en ce que d'une manière continue, on évacue du pied de colonne de la colonne combinée de division et de rectification, rapporté à la charge, 5 à 50 % de préférence de 5 à 25 %.

13. Procédé selon la revendication 1, caractérisé en ce que le réacteur, dans lequel les produits provenant de la division, l'alcool provenant de la tête et l'évacuation du pied de colonne de la colonne combinée de division et de rectification d) sont mis à réagir en continu, se présente comme un réacteur tubulaire et est mis en fonctionnement à une température de 100 à 140°C, à une pression de 2 bars et pendant un temps de séjour de 1 à 4 heures de préférence 2 heures.
